(19) Europäisches Patentamt
European Patent Office
Office européen des brevets

(11) Veröffentlichungsnummer: **0 062 825**
**B1**

(12) # EUROPÄISCHE PATENTSCHRIFT

(45) Veröffentlichungstag der Patentschrift: **11.03.87**

(51) Int. Cl.[4]: **C 07 D 251/48**, C 09 B 44/02, C 09 B 62/08

(21) Anmeldenummer: **82102617.6**

(22) Anmeldetag: **29.03.82**

(54) **Wasserlösliche Triazinverbindungen, ihre Herstellung und ihre Verwendung.**

(30) Priorität: **08.04.81 DE 3114087**

(43) Veröffentlichungstag der Anmeldung: **20.10.82 Patentblatt 82/42**

(45) Bekanntmachung des Hinweises auf die Patenterteilung: **11.03.87 Patentblatt 87/11**

(84) Benannte Vertragsstaaten: **CH DE FR GB LI**

(56) Entgegenhaltungen:
**EP - A - 0 022 209**
**DE - A - 2 915 323**
**GB - A - 951 667**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Nickel, Horst, Dr., Fontanestrasse 23, D-5090 Leverkusen 1 (DE)**
Erfinder: **Wild, Peter, Dr., Hainstrasse 7, D-6305 Alten Buseck (DE)**
Erfinder: **Stöhr, Frank-Michael, Dr., Weidenweg 25, D-5093 Burscheid (DE)**

**EP 0 062 825 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind wasserlösliche Triazinverbindungen, die in ihrer Betainform der Formel (I)

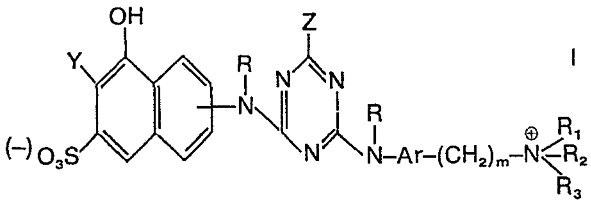

I

entsprechen, worin

Y für Wasserstoff oder den Rest D–(N=N–M)$_m$–N=N–

D für den Rest einer Diazokomponente der Benzol-, Naphthalin- oder heterocyclischen Reihe,

M für den Rest einer Kupplungskomponente der Benzol- oder Naphthalin-Reihe,

R für Wasserstoff oder Methyl,

Ar für Phenylen oder Naphthylen,

Z für Halogen, Hydroxy, $C_1$–$C_4$-Alkoxy, $C_1$–$C_4$-Alkyl, Phenyl oder eine Gruppe der Formel (IV) oder (V)

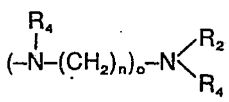

oder

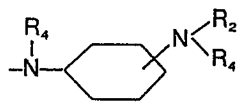

IV V

$R_1$, $R_2$ und $R_3$ für Wasserstoff, $C_1$ bis $C_4$-Alkyl, $C_3$- oder $C_4$-Alkenyl, Benzyl oder Phenylethyl, die durch Hydroxy, $C_1$- bis $C_4$-Alkoxy, Halogen oder Cyan und der Benzyl- und Phenylethylrest zusätzlich durch $C_1$–$C_4$-Alkyl substituiert sein können, oder

$R_2$ und $R_3$ zusammen mit dem Stickstoffatom, an das sie gebunden sind, für einen 5- oder 6-gliedrigen Ring,

$R_4$ für $R_3$ und ausserdem für gegebenenfalls durch Halogen, $C_1$–$C_4$-Alkyl, $C_1$–$C_4$-Alkoxy, Hydroxy oder die Reste (VI), (VII), (VIII)

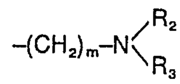

,

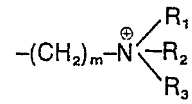

An$^{\ominus}$

VI VII

oder

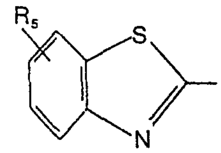

VIII

substituiertes Phenyl oder Naphthyl, Benzthiazolyl (2) oder Isobenzthiazolyl (3),

$R_5$ für Wasserstoff, Methyl, Methoxy oder Halogen,

An$^{\ominus}$ für ein Anion,

m für 0 oder 1,

n für 2 oder 3 und zusätzlich für 0, wenn o = 1 ist, und

o für 0, 1 oder 2 stehen,

worin die Summe der basischen und kationischen Gruppen grösser ist als die Anzahl der Sulfonsäuregruppen, ihre Herstellung und – wenn Y für den Rest D–(N=N–M)$_m$–N=N– steht – ihre Verwendung zum Färben von synthetischen und natürlichen Materialien, insbesondere Papier, sowie – wenn Y Wasserstoff bedeutet – ihre Verwendung als Kupplungskomponente zur Herstellung von Azofarbstoffen.

Aus der DE-A-29 15 323 sind basische bzw. kationische sulfonsäuregruppenhaltige Azofarbstoffe und ihre Verwendung zum Färben von Papier bekannt. Die neuen Farbstoffe unterscheiden sich von diesen Farbstoffen durch den

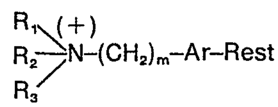

am Aminorest des Triazinringes.

Bevorzugt sind Farbstoffe der Formel (I), worin R für Wasserstoff steht.

Hervorzuheben aus den Farbstoffen (I) sind wasserlösliche, kationische Azofarbstoffe der Formel (II)

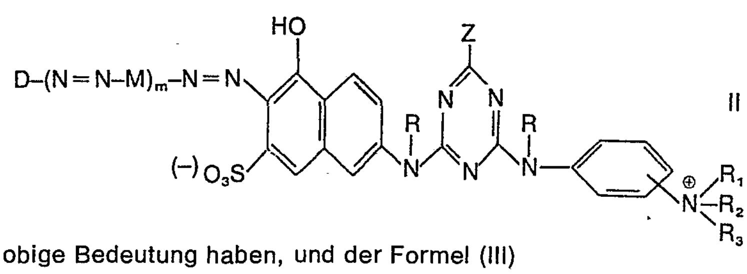

II

worin die Symbole die obige Bedeutung haben, und der Formel (III)

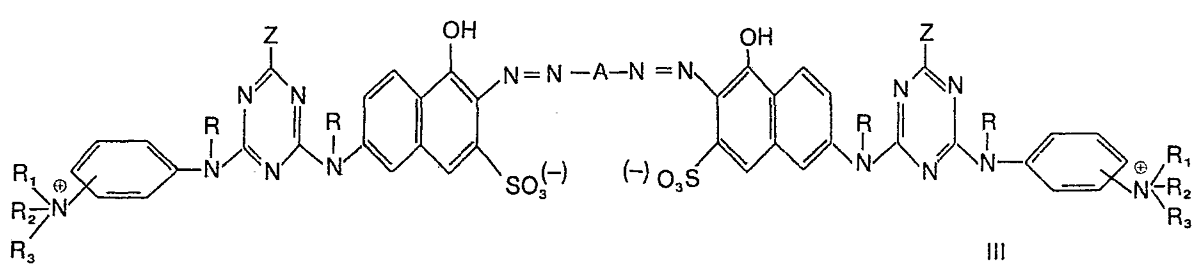

III

worin

A den Rest einer aromatischen Tetrazokomponente darstellt.

Um die Wasserlöslichkeit der Farbstoffe zu erreichen, ist es erforderlich, dass die Summe der basischen und kationischen Gruppen grösser als die Anzahl der Sulfonsäuregruppen ist, da jede Sulfonsäuregruppe mit jeweils einer basischen oder kationischen Gruppe ein inneres, schwerlösliches Salz bilden kann.

Dabei kann (können) die zusätzliche(n) basische(n) oder kationische(n) Gruppe(n) in D, M, A und Z lokalisiert sein.

Halogen steht insbesondere für Fluor, Chlor oder Brom.

D und M stehen bevorzugt für einen Rest der Benzol- oder Naphthalinreihe. Besonders geeignete Substituenten dieser Ringe sind $C_1$–$C_4$-Alkyl, Hydroxy, $C_1$–$C_4$-Alkoxy, Halogen, die Sulfonsäuregruppe, die Gruppen (VI)–(VIII) und eine Gruppe der Formel (IX)

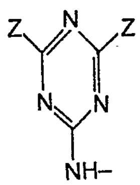

IX

Bevorzugte Reste A sind gegebenenfalls durch $C_1$–$C_4$-Alkyl, Hydroxy, $C_1$–$C_4$-Alkoxy oder Halogen substituiertes 1,3- oder 1,4-Phenylen oder ein Rest der Formel (X)

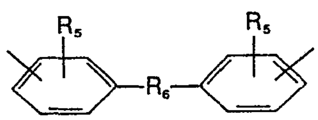

X

mit $R_6$ = direkte Bindung, $-(CH_2)_p-$, $-O-$, $-O-(CH_2)_p-O-$, $-SO_2-$, $-NH-CO-$, $-NH-CO-NH-$, $-NH-CO-(CH_2)_p-CO-NH-$, $-CO-NH-(CH_2)_p-NH-CO-$ oder

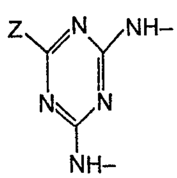

XI

und p = 1, 2 oder 3.

Die Auswahl der Diazo- bzw. Tetrazokomponenten muss in der Weise erfolgen, dass die Bedingung der Wasserlöslichkeit erfüllt wird, d.h., dass das entstehende Farbstoffmolekül mindestens eine basische oder kationische Gruppe mehr enthält als das Molekül Sulfonsäuregruppen besitzt.

Besitzt also die Kupplungskomponente I (Y = Wasserstoff) neben der kationischen Gruppe in Z eine zweite basische oder kationische Gruppe, so kann eine aromatische carbocyclische oder heterocyclische Diazo- bzw. Tetrazokomponente – die frei ist von anionischen Gruppen – wie beispielsweise Anilin, Aminoazobenzol, Aminonaphthalin, 4,4'-Diamino-benzoyl-anilid, 4,4'-Diamino-3,3'-dimethyl- bzw. dimethoxydiphenyl, 4,4'-Diaminodiphenyl-ethan (1, 2) Verwendung finden.

Besitzt die Kupplungskomponente I (Y = Wasserstoff) dagegen nur die kationische Gruppe, so muss die aromatische carbocyclische oder heterocyclische Diazo- oder Tetrazokomponente mindestens eine basische oder kationische Gruppe aufweisen, um die Wasserlöslichkeit zu erreichen.

Geeignete Diazokomponenten dieser Art sind beispielsweise: Anilin-3- oder -4-trimethylammoniumchlorid, -sulfat, -methosulfat, -tosylat, -benzol-sulfonat, 3- oder 4-Amino-benzyldi- oder -trimethylammoniumchlorid, -methosulfat oder 2-Aminonaphthalin-5-methylentrimethylammonium-methosulfat.

Hierzu zählen auch Amino-azo-Verbindungen mit gegebenenfalls über eine Methylengruppe gebundenen Ammoniumgruppen wie 4-Aminoazobenzol-4'-dimethylammonium- oder -trimethylammonium-chlorid, 4-Amino-azobenzol-3'-trimethylammonium-chlorid, 4-Amino-azobenzol-4'- bzw. 3'-methylentrimethylammonium-chlorid und im Benzolkern dieser Azobenzole in 3- oder 2-Stellung durch Methyl, Methoxy, Ethoxy, Chlor substituierte Verbindungen wie 2-Aminonaphthalin-5-methylen-trimethylammonium-chlorid → Anilin bzw. → 3-Methylanilin bzw. → 2-Methoxyanilin.

Die Kupplungskomponenten I (Y = H) werden in bekannter Weise im allgemeinen folgendermassen hergestellt: Die Naphthalin-sulfonsäure (XII) wird zunächst mit Cyanurhalogenid, vorzugsweise -chlorid oder -fluorid, zur 1:1-Verbindung umgesetzt. Anschliessend wird durch Halogenaustausch beispielsweise mit Ammoniak oder Aminen HZ der Z-Substituent eingeführt.

Nach Einführung des Substituenten Z in der zweiten Stufe (bei 20–50 °C) wird in dritter Stufe bei 70–95 °C die Kondensation mit der Aminoverbindung (XIII) vorgenommen.

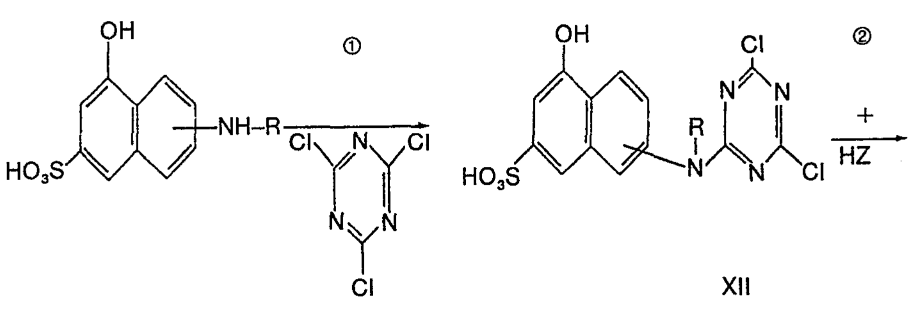


XII

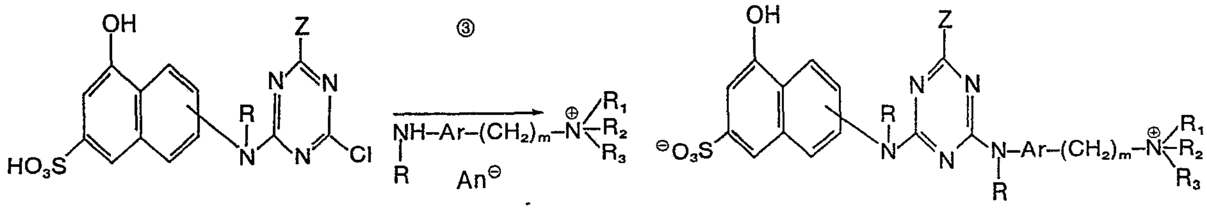

XIII I (Y=H)

Falls Z = F, Cl, Br wird die, die Ammoniumgruppe tragende aromatische Amino-Verbindung (XIII) in zweiter Stufe kondensiert.

Selbstverständlich können die einzelnen Kondensationsschritte vertauscht werden und beispielsweise zuerst die Amino-Verbindung (XIII) mit dem Cyanurhalogenid kondensiert werden.

Als Beispiele für die Verbindungen HZ seien genannt:
$C_1$–$C_4$-Mono- bzw. Dialkylamine wie Methyl-, Ethyl-, Chlorethyl-, Propyl-, iso-Propyl-, Butyl-, Hydroxy-ethyl, 2-Methoxy-ethyl-, Dimethyl-, Diethyl-, Di-i-propyl-, Di-n-propyl-, Diethanol-, Di-iso-propanol-, Di-iso-butyl-, Methyl-ethyl-, Methyl-ethanol- und Ethyl-ethanolamin, 3-Methylaminopropanitril,
Cycloalkylamine wie
Piperidin, Morpholin, Piperazin, Methyl-piperazin, Hydroxy-ethylpiperazin,
Aralkylamine wie
Benzylamin, Benzyl-methylamin, Benzylethanolamin,
die im Phenylkern durch Chlor oder Methyl substituiert sein können. Diamine wie
Ethylendiamin, N,N'-Dimethylethylendiamin, 1-Amino-1-diethylaminoethan, Propylendiamin, N-Methyl- und N,N-Dimethyl- oder ethyl-propylendiamin, Dipropylentriamin, Diethylentriamin, N,N',N''-Trimethyldiethylentriamin, 1,4-Diaminocyclohexan.

Aromatische Amine wie Anilin, N-Methyl oder Ethyl- oder Hydroxyethyl-anilin, die im Phenylkern in o-, m- oder p-Stellung durch
Methyl, Ethyl, Chlor, Methoxy, Ethoxy,
N,N-Dimethyl- oder N,N-Diethyl-amino,
Trimethylammoniumchlorid, -methosulfat oder -acetat,
Methylendimethylamino oder Methylentrimethylammonium-methosulfat
substituiert sein können,
1-Naphthylamin, Dehydrothiotoluidin oder -xylidin, 2-Amino-benzo-thiazol,
3-Aminoisobenzothiazol,
2-Aminonaphthalin-5-methylentrimethylammoniumchlorid
ferner Methyl- und Dimethylhydrazin.

Die neuen Azofarbstoffe I (Y = Rest eines Azofarbstoffs) werden nach üblichen in der Azochemie bekannten Verfahren hergestellt (siehe z.B. Houben-Weyl, Methoden der organischen Chemie Band X/3, Georg Thieme Verlag Stuttgart 1965 ab Seite 226 und Seite 270) durch Azokupplung von Diazonium- bzw. Tetrazoniumverbindungen mit den Kupplungskomponenten der Formel I (X = H) vorzugsweise in wässrigem Medium.

Falls Z = Alkyl oder Aryl, sind natürlich nur 2 austauschbare Halogen-Substituenten im Triazinring zur Verfügung, wobei wahlweise zuerst (XIII) oder die Aminonaphtholsulfonsäure kondensiert werden kann.

Geeignete Aminoverbindungen (XIII) sind beispielsweise: (wobei als Anion $An^{\ominus}$ vorzugsweise Chlorid, Bromid, Methosulfat, Tosylat, Benzolsulfonat in Betracht kommen)

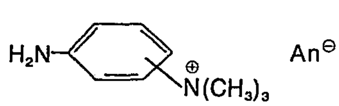

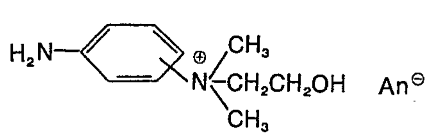

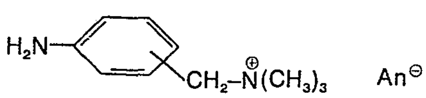

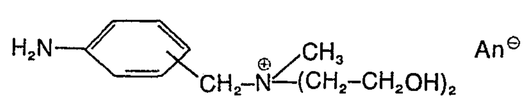

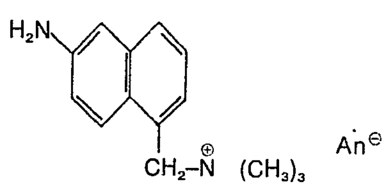

Die Farbstoffe werden zum Färben von mit kationischen Farbstoffen färbbaren Materialien verwendet. Genannt seien beispielsweise: Polyacrylnitril, sauer modifizierte Polyester, z.B. Polyglykolterephthalate, wie sie in der belgischen Patentschrift 549 179 oder US-Patentschrift 2 893 816, beschrieben werden, sauer modifizierte Polyamide, tannierte vegitabilische Fasern (Baumwolle), Leder und vorzugsweise Papier. Geeignet sind die Farbstoffe zum Färben von geleimtem sowie umgeleimten Papier, wobei von gebleichtem oder ungebleichtem Zellstoff ausgegangen werden kann und Laub- oder Nadelholz-Zellstoff wie Birken- und/oder Kiefern-Sulfit und/oder Sulfat-Zellstoff verwendet werden kann.

Die Farbstoffe werden sowohl als Pulver- bzw. Granulat-Präparationen als auch in Form von konzentrierten Lösungen zum Einsatz gebracht. Pulver-Präparationen werden in üblicher Weise mit Stellmaterialien wie Natriumsulfat, -phosphat, -chlorid, -acetat in Gegenwart von Entstaubungsmitteln eingestellt, oder die Farbstoffe werden direkt als Sprühtrockungspräparationen in den Handel gebracht. Konzentrierte Farbstofflösungen können wässriger oder wässrig/organischer Art sein, wobei übliche, umweltfreundliche und möglichst gut abbaubare Zusätze bevorzugt werden wie organische Säuren, vorzugsweise Essigsäure, Ameisensäure, Amide wie Formamid, Dimethylformamid, Harnstoff, Alkohole wie Glykol, Diglykol, Diglykolether, vorzugsweise dessen Methyl- oder Ethyl-ether.

Die Farbstoffe besitzen ein ausgezeichnetes Ziehvermögen und sehr gute Allgemein-Echtheiten. Papierfärbungen zeichnen sich durch sehr gute Nassechtheiten sowie Alaun-, Säure- und Alkali-Echtheiten aus. Sie besitzen eine überraschend hohe Lichtechtheit, bei gleichzeitig hoher Klarheit und Farbstärke.

Bei den in den Beispielen mit «I. Nr.» gekennzeichneten Nummern handelt es sich um Farbtonangaben nach «Colour Index Hue Indication Chart».

Beispiel 1

18,5 Teile (0,1 Mol) Cyanurchlorid, gelöst in 200 Teilen Aceton, werden in 300 Teilen Eis-Wasser suspendiert. Zu dieser Suspension gibt man bei 0 bis 5 °C als 1. Kondensationskomponente die wässrige Lösung von 18,6 Teilen (0,1 Mol) Anilin-3-trimethylammonium-chlorid (Volumen ca. 70 Teile) zu und rührt bis die Erstkondensation beendet (ca. ½ Stunde) ist, wobei man pH 4–5 hält und die freiwerdende Säure mit 20%iger Natriumcarbonatlösung neutralisiert. Das Kondensationsprodukt ist ausgefallen.

Anschliessend gibt man als 2. Kondensationskomponente die Lösung von 18 Teilen Anilin-3-trimethylammonium-chlorid (Volumen ca. 70 Teile) zu, erwärmt bis 40–50 °C, hält den pH-Wert bei 5–6 durch Zutropfen von 20%iger Natriumcarbonat-(Soda)-Lösung und rührt bis die Ammoniumverbindung nicht mehr nachweisbar ist (Prüfung durch Diazotierung und Kupplung auf R-Salz-Lösung). Die erhaltene Monochlorverbindung ist nach beendeter Kondensation in Lösung. Nun gibt man die Lösung von 21 Teilen 6-Amino-1-hydroxynaphthalin-3-sulfonsäure (I-Säure) als 3. Kondensationskomponente in 200 Teilen Wasser (pH ca. 6) zu, erwärmt auf 80–90 °C und neutralisiert freiwerdende Säure mit 20%iger Sodalösung, indem man pH ca. 6 hält.

Nach beendeter Drittkondensation liegt die Kupplungskomponente der Formel

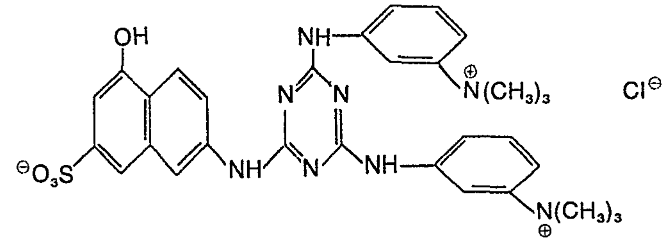

1,1

vor, die durch Ansäuren mit 28%iger Salzsäure auf pH 2 gestellt wird und in dieser Säure/Salz-Form direkt weiterverarbeitet werden kann oder in üblicher Weise z.B. als Zinkdoppelsalz isoliert wird.

Verwendet man die I-Säure als erste Kondensationskomponente, kondensiert wie oben bei 0 bis 5 °C mit Cyanurchlorid, dann führt man zweckmässig nach der 2. Kondensationsstufe bei 40–50 °C mit Anilin oder N-Methylanilin-, 2-Methylanilin, Methylamin, Dimethylamin, N,N-Dimethylpropylendiamin oder anderen oben angeführten Aminen HZ eine Zwischenisolierung durch und kondensiert schliesslich in dritter Stufe bei 80–95 °C mit Anilin-3-trimethylammoniumchlorid bzw. Acetat, z.B. zur Kupplungskomponente

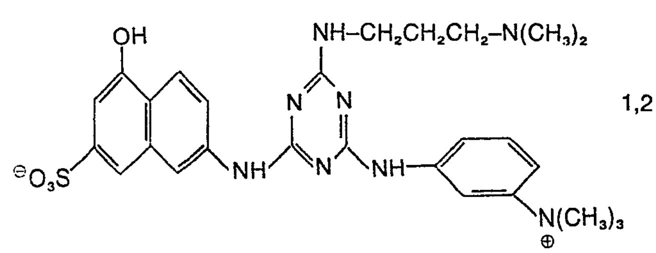

1,2

Kondensiert man in dritter Stufe mit 3- oder 4-Aminobenzyl-trimethyl-ammoniumchlorid, so erhält man z.B. die Kupplungskomponente

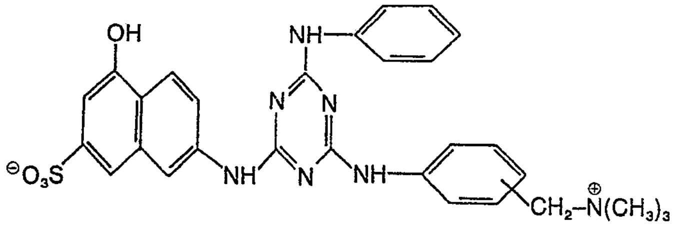
1,3

In der folgenden Tabelle sind weitere Kupplungskomponenten (I) (Y = H) angeführt.

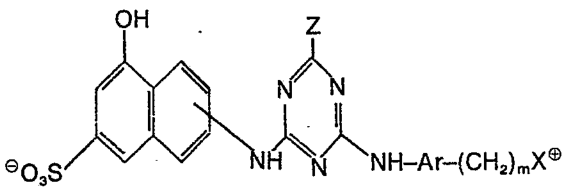

| | Säure | Hz | $H_2N-Ar-(CH_2)_m-N^{\oplus}(R_1)(R_2)(R_3)$ $An^{\ominus}$ |
|---|---|---|---|
| 1,4 | I-Säure | Anilin | 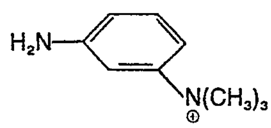 |
| 1,5 | " | N-Methylanilin | " |
| 1,6 | " | Methylamin | " |
| 1,7 | " | Dimethylamin | " |
| 1,8 | " | Diethanolamin | " |
| 1,9 | " | Diisopropanolamin | " |
| 1,10 | " | Morpholin | " |
| 1,11 | " | Piperidin | " |
| 1,12 | " | Piperazin | " |
| 1,13 | " | N-Me-Piperazin | " |
| 1,14 | " | N-Hydroxyethylpiperazin | " |
| 1,15 | " | Ammoniak | " |
| 1,16 | " | Ethylendiamin | " |
| 1,17 | " | Dimethylamin | 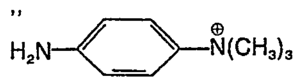 |
| 1,18 | " | Diethanolamin | " |
| 1,19 | I-Säure | Diethanolamin | 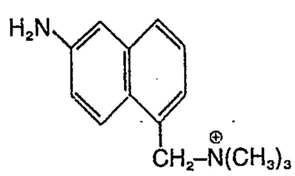 |
| 1,20 | " | Diisopropanolamin | 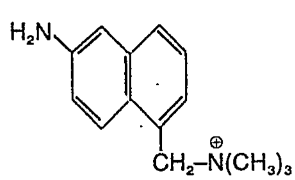 |
| 1,21 | " | Ammoniak | " |
| 1,22 | " | " | 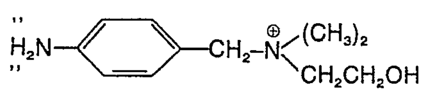 |
| 1,23 | " | Diethanolamin | " |

| | Säure | Hz | 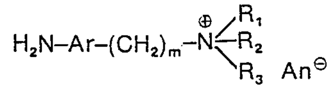 |
|---|---|---|---|
| 1,24 | I-Säure | Diethanolamin | 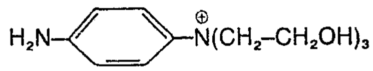 |
| 1,25 | Methyl-I-Säure* | Dimethylpropylendiamin | 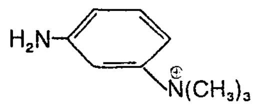 |
| 1,26 | Gamma-Säure** | Dimethylamin | " |
| 1,27 | " | Diethanolamin | " |
| 1,28 | " | Dimethylpropylendiamin | " |
| 1,29 | " | 4-Acetylaminoanilin | " |
| 1,30 | I-Säure | Aminoethylpiperazin | 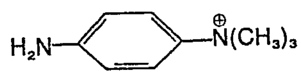 |
| 1,31 | " | " | |

* 6-Methylamino-1-hydroxynaphthalin-3-sulfonsäure

** 7-Amino-1-hydroxynaphthalin-3-sulfonsäure

Beispiel 2

Die salzsaure Lösung von 25 Teilen (0,1 Mol) 2-Aminonaphthalin-5-methylentrimethylammonium-chlorid (Volumen ca. 70 Teile) wird in üblicher Weise nach Zugabe von etwa 100 Teilen Eis mit 24 Teilen 30%iger wässriger Natriumnitrit-Lösung bei 0 °C diazotiert. Das Diazoniumsalz bleibt in Lösung. Kurz vor dem Kuppeln wird überschüssige salpetrige Säure mit Amidosulfonsäure zerstört.

60,7 Teile (0,1 Mol) der Kupplungskomponente 1,5 der Formel

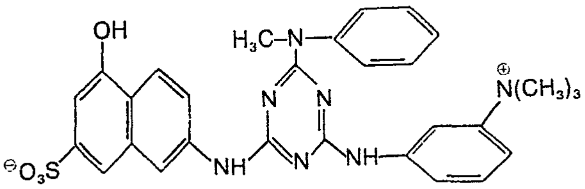

werden natronalkalisch in 700 Teilen Wasser gelöst und mit 50%iger Essigsäure auf pH 4,5 gestellt, wobei die Verbindung ausfällt. Man kühlt auf 5 °C ab und gibt dann die eiskalte Diazoniumsalz-Lösung zu. Durch Zutropfen von Natriumacetat-Lösung (20%ig) wird auf pH 4–5 gestellt und über Nacht gerührt bis die Kupplung beendet ist, wobei der Farbstoff ausfällt.

Der ausgefallene Farbstoff wird isoliert und getrocknet. Er stellt dann ein dunkles Pulver dar, das sich in Wasser mit roter Farbe löst.

Zur Herstellung einer Farbstoff-Lösung wird der ausgefallene Farbstoff des Kupplungs-Ansatzes mit 40%iger Natronlauge auf pH 12 gestellt, der Farbstoff isoliert und die erhaltene Farbstoffpaste in 120 Teilen 50%iger Essigsäure bei etwa 50 °C gelöst und von geringfügigen Rückständen geklärt.

Die erhaltene, kältestabile rote Farbstofflösung färbt Papier nach den üblichen Färbeverfahren (mit oder ohne Leimzusatz) in intensiven roten Tönen (I. Nr. 7). Der Farbstoff besitzt gute Nassechtheiten.

Verwendet man als Diazokomponente anstelle obiger 2-Amino-naphthalinammoniumverbindung die äquimolare Menge an Anilin-3-trimethylammoniumchlorid oder -4-trimethylammonium-chlorid so werden orangefarbene Farbstoffe bzw. Farbstoff-Lösungen erhalten (I. Nr. 5). Mit 3- oder 4-Aminobenzyl-trimethylammoniumchlorid als Diazokomponente werden ebenfalls orangefarbene Färbungen auf Papier erhalten (I. Nr. 5). 4-Aminoazobenzol-4′-trimethylammoniumchlorid als Diazokomponente ergibt einen blaustichig roten Farbstoff (I. Nr. 10). Setzt man als Kupplungskomponente anstelle obiger Verbindung 1,5 die Kupplungskomponenten 1,2 oder 1,3 1,8, 1,9, 1,10, 1,11, 1,14, 1,15, 1,19, 1,20, 1,27, 1,28, 1,30, 1,31, ein, so erhält man mit den obigen Diazokomponenten Farbstoffe, die Papier in ähnlichen Tönen färben wie mit obiger Kupplungskomponente 1,5.

Beispiel 3

Die salzsaure Lösung von 0,1 Mol der Kupplungskomponente der Formel 1.1 (Volumen 500 Teile) wird mit Eis auf 0° abgekühlt und mit der in üblicher Weise bereiteten Lösung von 0,1 Mol 4-Chlorbenzoldiazoniumchlorid (Volumen 230 Teile) vereinigt. Man tropft dann 20%ige Natrium-Acetat-Lösung zu bis pH 4–5 und rührt bis die Kupplung beendet ist. Der Farbstoff der Formel

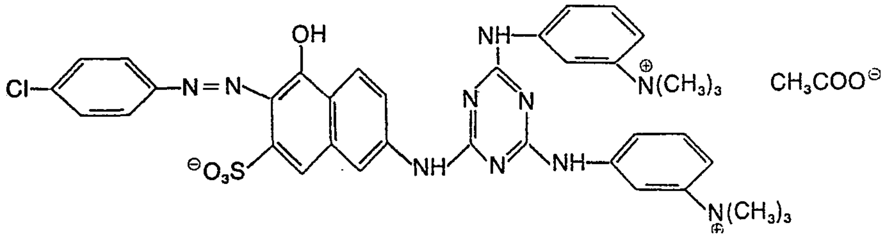

wird in üblicher Weise isoliert und entweder getrocknet oder mit 50%iger Essigsäure eine Farbstoff-Lösung hergestellt. Der Farbstoff färbt Papier in orangefarbenen Tönen (I. Nr. 6).

Verwendet man als Diazokomponente anstelle von
4-Chloranilin Anilin, 4-Aminotoluol,
2,4- oder 2,5-Dichloranilin oder
Anilin-3-trimethyl-ammonium-chlorid,
so werden orangefarbene Farbstoffe erhalten (I. Nr. 5–6). Mit den Diazokomponenten
4-Methoxyanilin, 2-Methoxyanilin,
4-Ethoxyanilin (I. Nr. 8),
2-(4-Aminophenyl)-6-methylbenzthiazol,
2-(4-Aminophenyl)-4,6-dimethylbenzthiazol,
2-(4-Aminophenyl)-benzimidazol (I. Nr. 7) oder
2-Aminonaphthalin-5-methylentrimethylammoniumchlorid (I. Nr. 7)
werden Farbstoffe erhalten, die Papier in intensiven roten Tönen färben. Unter Verwendung der Diazokomponenten
4-Aminoazobenzol oder 2-Methyl-4-Aminoazobenzol
oder 2'-Methoxy-4-aminoazobenzol
oder 4-Aminoazobenzol-4'-trimethylammoniumchlorid oder
2-Methyl-4-amino-benzol-azo-2'-naphthalin-5-methylentrimethylammoniumchlorid
werden Disazofarbstoffe erhalten, die Papier in blaustichig roten Tönen färben (I. Nr 10).

Verwendet man als Diazokomponente die Bis-Diazokomponente 4,4'-Diamino-benzoylanilid so entsteht ein roter Farbstoff (I. Nr. 9).

Unter Verwendung der Kupplungskomponenten 1,2, 1,12, 1,13, 1,14, 1,30 oder 1,31 werden bei Einsatz der obigen Diazokomponenten in den Farbtönen ähnliche Farbstoffe erhalten wie oben mit der Kupplungskomponente 1,1 beschrieben.

**Patentansprüche**

1. Triazinverbindungen, die in ihrer Betainform der Formel (I)

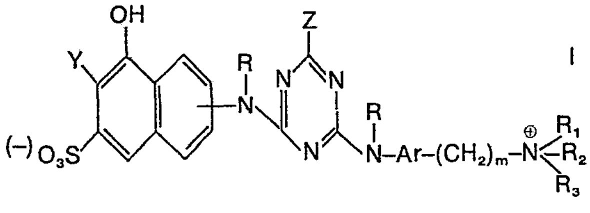

I

entsprechen, worin

Y für Wasserstoff oder den Rest $D-(N=N-M)_m-N=N-$

D für den Rest einer Diazokomponente der Benzol-, Naphthalin- oder heterocyclischen Reihe,

M für den Rest einer Kupplungskomponente der Benzol- oder Naphthalin-Reihe,

R für Wasserstoff oder Methyl,

Ar für Phenylen oder Naphthylen,

Z für Halogen, Hydroxy, $C_1$–$C_4$-Alkoxy, $C_1$–$C_4$-Alkyl, Phenyl oder eine Gruppe der Formel (IV) oder (V)

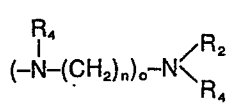 oder 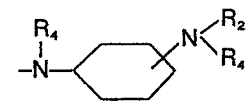

IV V

$R_1$, $R_2$ und $R_3$ für Wasserstoff, $C_1$ bis $C_4$-Alkyl, $C_3$- oder $C_4$-Alkenyl, Benzyl oder Phenylethyl, die durch Hydroxy, $C_1$- bis $C_4$-Alkoxy, Halogen oder Cyan und der Benzyl- und Phenylethylrest zusätzlich durch $C_1$–$C_4$-Alkyl substituiert sein können, oder

$R_2$ und $R_3$ zusammen mit dem Stickstoffatom, an das sie gebunden sind, für einen 5- oder 6-gliedrigen Ring,

$R_4$ für $R_3$ und ausserdem für gegebenenfalls durch Halogen, $C_1$–$C_4$-Alkyl, $C_1$–$C_4$-Alkoxy, Hydroxy oder die Reste (VI), (VII), (VIII)

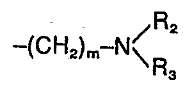 , 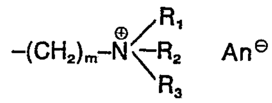

VI VII

oder 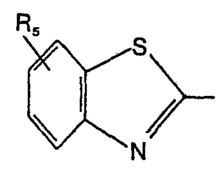

VIII

substituiertes Phenyl, oder Naphthyl, Benzthiazolyl (2) oder Isobenzthiazolyl (3),

$R_5$ für Wasserstoff, Methyl, Methoxy oder Halogen,

$An^{\ominus}$ für ein Anion,

m für 0 oder 1,

n für 2 oder 3 und zusätzlich für 0, wenn o = 1 ist, und

o für 0, 1 oder 2 stehen,

worin die Summe der basischen und kationischen Gruppen grösser ist als die Anzahl der Sulfonsäuregruppen.

2. Triazinverbindungen der Formel des Anspruchs 1, in der Y für Wasserstoff steht.

3. Triazinverbindungen gemäss Anspruch 1 der Formel (II)

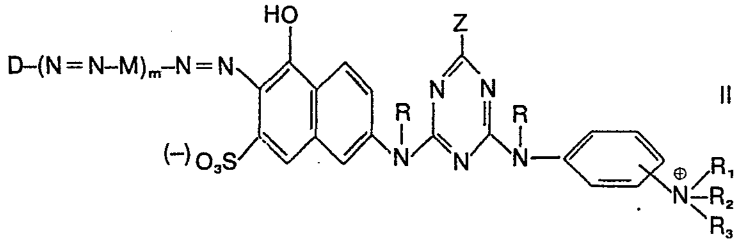

worin die Symbole die in Anspruch 1 angegebene Bedeutung haben.

4. Triazinverbindungen gemäss Anspruch 1 der Formel (III)

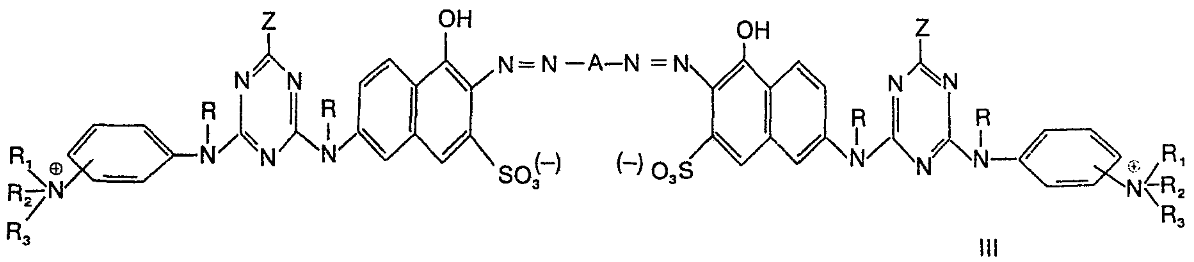

III

worin

A den Rest einer aromatischen Tetrazokomponente darstellt, und

Z, R, $R_1$, $R_2$ und $R_3$ die in Anspruch 1 angegebene Bedeutung haben.

5. Triazinverbindungen der Formel (II) des Anspruchs 3, worin D und M für einen Rest der Benzol- oder Naphthalinreihe stehen, die durch $C_1$–$C_4$-Alkyl, Hydroxy, $C_1$–$C_4$-Alkoxy, Halogen, eine Sulfonsäuregruppe oder eine Gruppe der Formel (VI)–(IX)

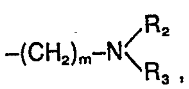 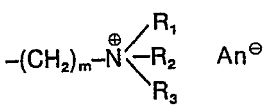 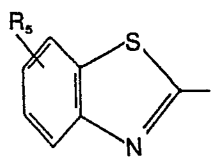 oder 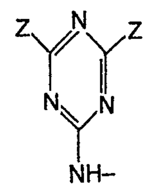 IX

VI VII VIII

substituiert sein können, worin

R, $R_1$, $R_2$, $R_3$, $R_5$, $An^{\ominus}$, Z und m die in Anspruch 1 angegebene Bedeutung haben.

6. Triazinverbindungen der Formel (III) des Anspruchs 4, worin

A für gegebenenfalls durch $C_1$–$C_4$-Alkyl, Hydroxy, $C_1$–$C_4$-Alkoxy oder Halogen substituiertes 1,3- oder 1,4-Phenylen oder einen Rest der Formel (X)

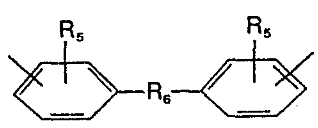 X

worin

$R_5$ für Wasserstoff, $C_1$–$C_4$-Alkyl, $C_1$–$C_4$-Alkoxy oder Halogen,

$R_6$ für eine direkte Bindung, $-(CH_2)_p-$, $-O-$, $-O-(CH_2)_p-O-$, $-SO_2-$, $-NH-CO-$, $-NH-CO-NH-$, $-NH-CO-(CH_2)_p-CO-NH-$, $-CO-NH-(CH_2)_p-NH-CO-$ oder einen Rest der Formel (XI)

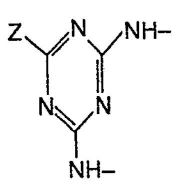 XI

und p für 1, 2 oder 3 stehen, und Z, R, $R_1$, $R_2$ und $R_3$ die Bedeutung des Anspruchs 1 hat.

7. Verfahren zur Herstellung von Triazinverbindungen des Anspruchs 1, dadurch gekennzeichnet, dass man Verbindungen der Formel

$$D-(N=N-M)_m-NH_2$$

diazotiert und mit Verbindungen der Formel

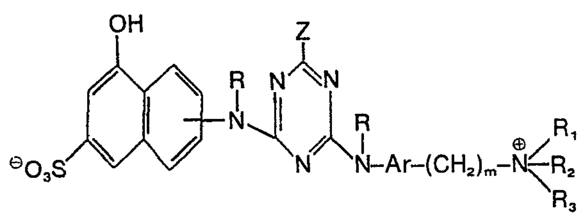

worin

die Symbole die in Anspruch 1 angegebene Bedeutung haben, kuppelt.

8. Verfahren zur Herstellung von Triazinverbindungen des Anspruchs 2, dadurch gekennzeichnet, dass man Cyanurhalogenid in beliebiger Reihenfolge mit Verbindungen der Formeln

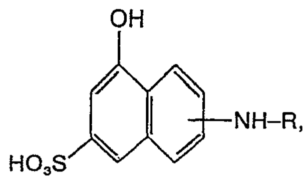

ZH und

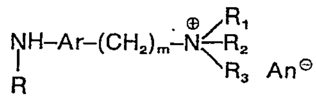

worin die Symbole die in Anspruch 1 angegebene Bedeutung haben, und An$^{\ominus}$ für ein Anion steht, umsetzt.

9. Verfahren zum Färben von natürlichen und synthetischen kationisch anfärbbaren Substraten und Massen, dadurch gekennzeichnet, dass man Farbstoffe des Anspruchs 1 worin Y den Rest $D-(N=N-M)_m-N=N-$ bedeutet verwendet.

10. Verfahren zum Färben von Papier, dadurch gekennzeichnet, dass man Farbstoffe des Anspruchs 1 worin Y den Rest $D-(N=N-M)_m-N=N-$ bedeutet verwendet.

## Claims

1. Triazine compounds which in their betaine form correspond to the formula (I)

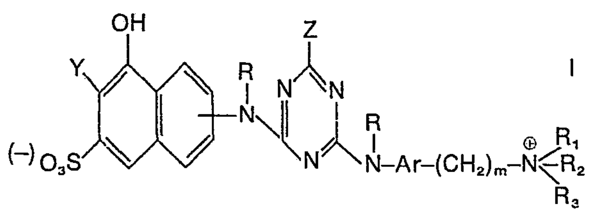

I

wherein

Y represents hydrogen or the radical $D-(N=N-M)_m-N=N-$,

D represents the radical of a diazo component of the benzene, naphthalene or heterocyclic series,

M represents the radical of a coupling component of the benzene or naphthalene series,

R represents hydrogen or methyl,

Ar represents phenylene or naphthylene,

Z represents halogen, hydroxyl, $C_1$–$C_4$-alkoxy, $C_1$–$C_4$-alkyl, phenyl or a group of the formula (IV) or (V)

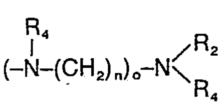

IV

or

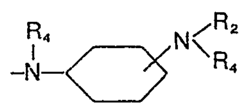

V

$R_1$, $R_2$ and $R_3$ represent hydrogen, $C_1$ to $C_4$-alkyl, $C_3$- or $C_4$-alkenyl, benzyl or phenylethyl, which can be substituted by hydroxyl, $C_1$–$C_4$-alkoxy, halogen or cyano and the benzyl and phenylethyl radicals can additionally be substituted by $C_1$–$C_4$-alkyl, or $R_2$ and $R_3$, together with the nitrogen atom to which they are bonded, represent a 5- or 6-membered ring,

$R_4$ represents $R_3$ and, in addition, phenyl which is optionally substituted by halogen, $C_1$–$C_4$-alkyl, $C_1$–$C_4$-alkoxy, hydroxyl or the radicals (VI), (VII) or (VIII)

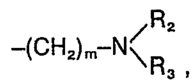

VI

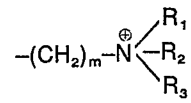

VII

or

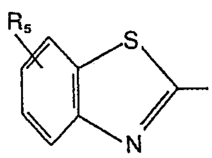

VIII

or naphthyl, 2-benzothiazolyl or 3-isobenzothiazolyl,

$R_5$ represents hydrogen, methyl, methoxy or halogen,

An$^{\ominus}$ represents an anion,

m represents 0 or 1,

n represents 2 or 3 and, in addition, 0 when o = 1, and

o represents 0, 1 or 2,

the sum of the basic and cationic groups being larger than the number of sulphonic acid groups.

2. Triazine compounds of the formula of Claim 1, in which Y represents hydrogen.

3. Triazine compounds according to Claim 1 of the formula (II)

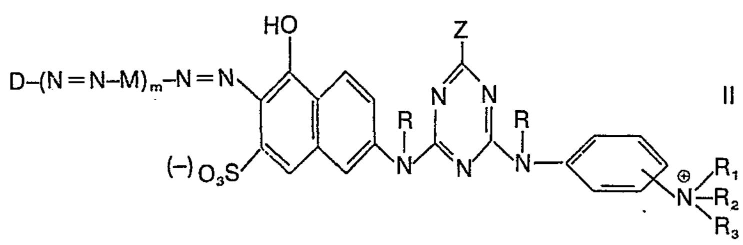

II

wherein the symbols have the meaning given in Claim 1.

4. Triazine compounds according to Claim 1 of the formula (III)

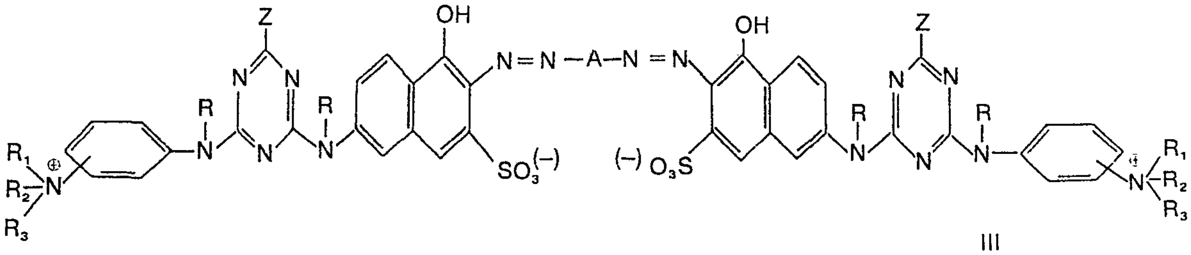

III

wherein

A represents the radical of an aromatic tetraazo component and

Z, R, $R_1$, $R_2$ and $R_3$ have the meaning given in Claim 1.

5. Triazine compounds of the formula (II) of Claim 3,

wherein

D and M represent a radical of the benzene or naphthalene series which can be substituted by $C_1$–$C_4$-alkyl, hydroxyl, $C_1$–$C_4$-alkoxy, halogen, a sulphonic acid group or a group of the formula (VI)–(IX)

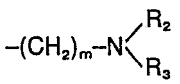

VI

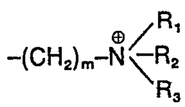 An$^{\ominus}$

VII

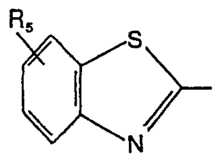

VIII

or

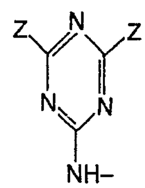

IX

wherein

R, $R_1$, $R_2$, $R_3$, $R_5$, An$^{\ominus}$, Z and m have the meaning given in Claim 1.

and

p represents 1, 2 or 3, and

Z, R, $R_1$, $R_2$ and $R_3$ have the meaning of Claim 1.

6. Triazine compounds of the formula (III) of Claim 4, wherein

A represents 1,3- or 1,4-phenylene optionally substituted by $C_1$–$C_4$-alkyl, hydroxyl, $C_1$–$C_4$-alkoxy or halogen, or a radical of the formula (X)

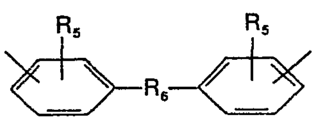

X

wherein

$R_5$ represents hydrogen, $C_1$–$C_4$-alkyl, $C_1$–$C_4$-alkoxy or halogen,

$R_6$ represents a direct bond, $-(CH_2)_p-$, –O–, $-O-(CH_2)_p-O-$, $-SO_2-$, –NH–CO–, –NH–CO–NH–, $-NH-CO-(CH_2)_p-CO-NH-$, $-CO-N-H-(CH_2)_p$ –NH–CO– or a radical of the formula (XI)

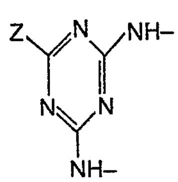

XI

7. Process for the preparation of triazine compounds of Claim 1, characterised in that compounds of the formula

$$D-(N=N-M)_m-NH_2$$

are diazotised and the product is coupled with compounds of the formula

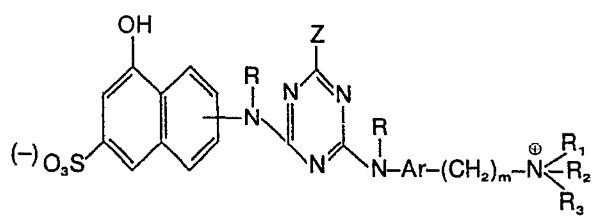

wherein

the symbols have the meaning given in Claim 1.

8. Process for the preparation of triazine compounds of Claim 2, characterised in that cyanuric halide is reacted in any desired sequence with compounds of the formula

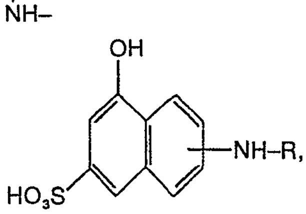, ZH and 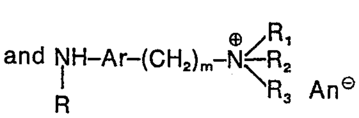

wherein

the symbols have the meaning given in Claim 1 and An$^{\ominus}$ represents an anion.

9. Process for dyeing natural and synthetic cationically dyeable substrates and compositions, characterised in that dyestuffs of Claim 1, wherein Y denotes the radical $D-(N=N-M)_m-N=N-$, are used.

10. Process for dyeing paper, characterised in that dyestuffs of Claim 1, wherein Y denotes the radical $D-(N=N-M)_m-N=N-$, are used.

**Revendications**

1. Composés triaziniques qui, sous leur forme de bétaïne, répondent à la formule (I)

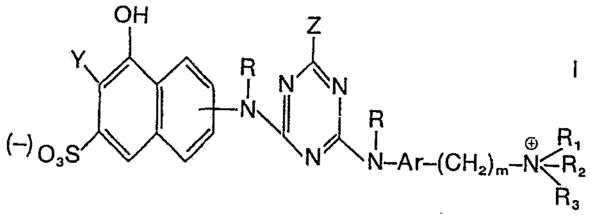

I

dans laquelle

Y représente un atome d'hydrogène ou le reste $D-(N=N-M)_m-N=N-$

D représente le reste d'un composant diazotable de la série benzénique, naphtalénique ou hétérocyclique,

M représente le reste d'un copulant de la série benzénique ou naphtalénique,

R représente un atome d'hydrogène ou un groupe méthyle,

Ar représente un reste phénylène ou naphtylène,

Z représente un atome d'halogène, un groupe hydroxy, alcoxy en $C_1-C_4$, alkyle en $C_1-C_4$, phényle ou un groupe de formule (IV) ou (V)

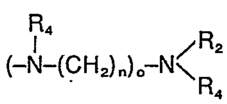

ou

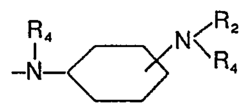

IV V

$R_1$, $R_2$ et $R_3$ représente chacun un atome d'hydrogène, un groupe alkyle en $C_1-C_4$, alcényle en $C_3$ ou $C_4$, benzyle ou phényléthyle, qui peuvent être substitués par un groupe hydroxy, par un groupe alcoxy en $C_1-C_4$, par un atome d'halogène ou un groupe cyano et les restes benzyle et phényléthyle pouvant en outre être substitués par un groupe alkyle en $C_1-C_4$, ou

$R_2$ et $R_3$ forment, avec l'atome d'azote auquel ils sont fixés, un noyau pentagonal ou hexagonal,

$R_4$ représente $R_3$ et peut représenter en outre un groupe phényle, ou naphtyle, benzothiazolyle (2) ou isobenzothiazolyle (3), éventuellement substitués par de l'halogène, par un groupe alkyle en $C_1-C_4$, alcoxy en $C_1-C_4$, hydroxy ou par les restes (VI), (VII), ou (VIII)

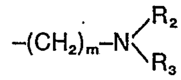

,

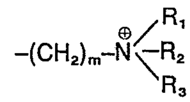

$An^{\ominus}$

IV V

ou

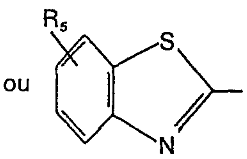

VII

$R_5$ représente un atome d'hydrogène, un groupe méthyle, méthoxy ou un atome d'halogène,

$An^{\ominus}$ représente un anion,

m vaut 0 ou 1,

n vaut 2 ou 3 et vaut en outre 0 quand o est nul, et

o vau 0, 1 ou 2,

la somme des groupes basiques et cationiques étant supérieure au nombre des groupes acides sulfoniques.

2. Composés triaziniques formule selon la revendication 1, dans laquelle Y représente un atome d'hydrogène.

3. Composés triaziniques selon la revendication 1, de formule (II)

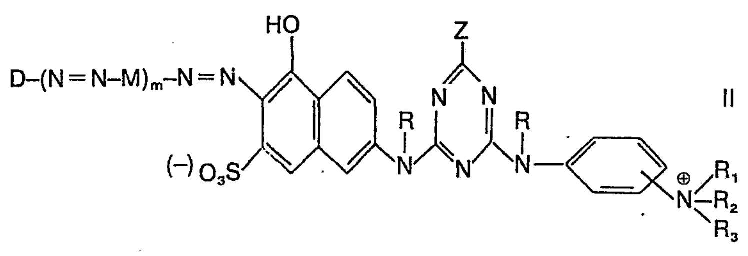

II

dans laquelle les symboles ont le sens indiqué à la revendication 1.

4. Composés triaziniques selon la revendication 1, de formule (III)

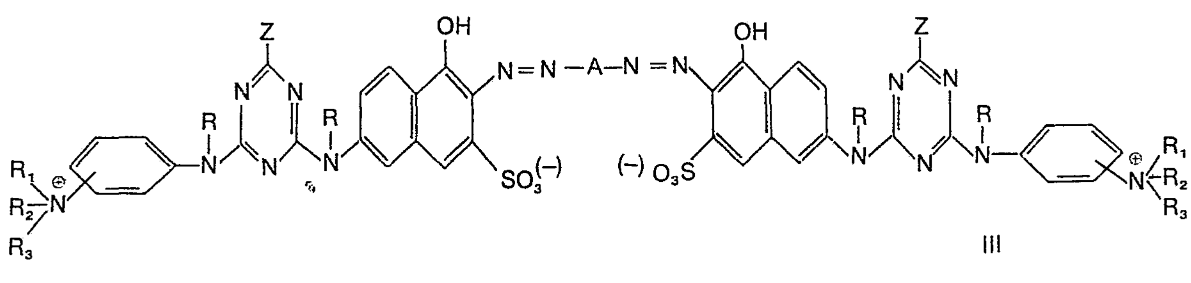

III

dans laquelle

A représente le reste d'un composant tétrazotable aromatique, et

Z, R, $R_1$, $R_2$ et $R_3$ ont le sens indiqué à la revendication 1.

5. Composés triaziniques de formule (II) selon la revendication 3, dans laquelle

D et M représentent un reste de la série benzénique ou naphtalénique, qui peut être substitué par un groupe alkyle en $C_1$–$C_4$, hydroxy, alcoxy en $C_1$–$C_4$, halogéno, un groupe acide sulfonique ou un groupe de formule (VI) à (IX)

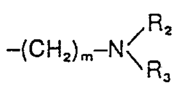

VI

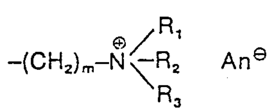

VII

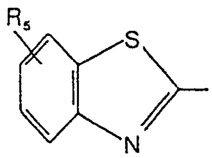

VIII

ou

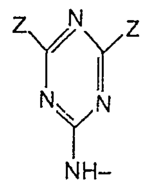

IX

dans lesquelles

R, $R_1$, $R_2$, $R_3$, $R_5$, $An^{\ominus}$, Z et m ont le sens indiqué à la revendication 1.

6. Composés triaziniques de formule (III) selon la revendication 4, dans lesquels: A représente un reste 1,3 ou 1,4-phénylène (éventuellement substitué par un groupe alkyle en $C_1$–$C_4$, hydroxy, alcoxy en $C_1$–$C_4$ ou halogéno) ou un reste de formule (X)

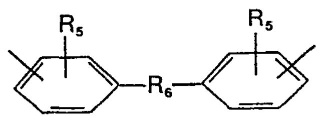

X

dans laquelle

$R_5$ représente un atome d'hydrogène, un groupe alkyle en $C_1$–$C_4$, alcoxy en $C_1$–$C_4$ ou halogéno,

$R_6$ représente une liaison directe, $-(CH_2)_p-$, $-O-$, $-O-(CH_2)_p-O-$, $-SO_2-$, $-NH-CO-$, $-NH-CO-NH-$, $-NHCO-(CH_2)_p-CO-NH-$, $-CO-NH-(CH_2)_p-NH-CO$ ou un reste de formule (XI)

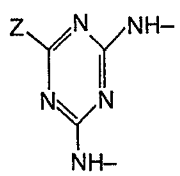

et p vaut 1, 2 ou 3, et Z, R, $R_1$, $R_2$ et $R_3$ ont le sens indiqué à la revendication 1.

7. Procédé pour préparer des composés triaziniques de la revendication 1, caractérisé en ce qu'on diazote des composés de formule:

$$D-(N=N-M)_m-NH_2$$

et l'on copule avec des composés de formule

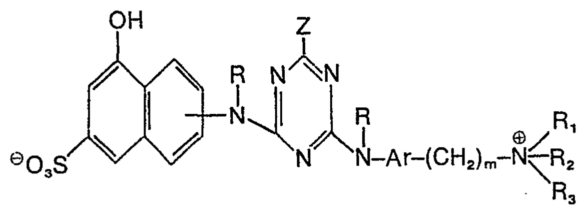

dans lesquelles

les symboles ont le sens indiqué à la revendication 1.

8. Procédé pour préparer des composés triaziniques selon la revendication 2, caractérisé en ce qu'on fait réagir un halogénure de cyanuryle, dans un ordre quelconque, avec les composés répondant aux formules

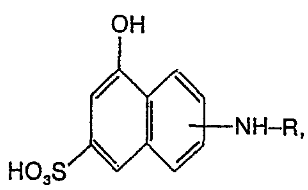

, ZH et

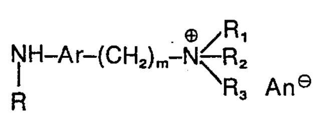

dans lesquelles

les symboles ont le sens indiqué à la revendication 1, et $An^{\ominus}$ représente un anion.

9. Procédé pour teindre ou colorer les substrats et compositions, naturels et synthétiques, colorables par des colorants cationiques, caractérisé en ce qu'on utilise des colorants selon la revendication 1, dans lesquels Y représente le reste $D-(N=N-M)_m-N=N-$.

10. Procédé pour colorer du papier, caractérisé en ce qu'on utilise des colorants selon la revendication 1, dans lesquels Y représente le reste $D-(N=N-M)_m-N=N-$.